# EUROPEAN PATENT APPLICATION

(11) **EP 2 389 957 A1**
(43) Date of publication of application: **30.11.2011**
(21) Application number: 10183952.0
(22) Date of filing: 16.12.2004
(51) Int. Cl.: A61L 15/46, A61L 15/20

(54) **Wound care product comprising a substance which inhibits the growth of bacteria in wounds**

(30) Priority: 17.12.2003 SE 0303400
(62) Divisional of application: 04809064.1
(71) Applicant: Mölnlycke Health Care AB, 402 52 Göteborg (SE)
(72) Inventor: Areskoug, Stefan, S-435 41, Mölnlycke (SE); Melhus, Åsa, S-222 40, Lund (SE)
(74) Representative: Olsson, Stefan

(57) **Abstract**

Wound care product which comprises a substance which inhibits the growth of bacteria in wounds. The wound care product comprises a layer which directly contacts the wound during use. The invention is characterized by the fact that the substance is xylitol. The wound care product comprises a gel into which xylitol has been introduced. The gel is arranged in said layer.

The invention also relates to the use of said substance in wound care products.

## Description

### TECHNICAL FIELD

The present invention relates to a wound care product which comprises a substance which inhibits the growth of bacteria in wounds. The invention also relates to a method for manufacturing the wound care product. The invention furthermore relates to the use, in wound care products, of a substance which inhibits the growth of bacteria in wounds. The invention additionally relates to the use of a substance for producing a composition which has a growth-inhibiting effect on bacteria in wounds.

### PRIOR ART

Wound care products exist in many different forms, such as ointments, pastes, dressings, plasters and bacteriostatic agents.

A wound-healing process can in principle be divided into three phases. The wound is first of all purged, after which tissue is regenerated, with this tissue being stabilized during a concluding maturation phase and becoming less fragile and more elastic.

During the regeneration phase, capillaries, fibroblasts and epithelium grow into the wound region and synthesize new tissue. The regenerated tissue is very fragile and sensitive to external influences. When treating wounds, dressings of some type are used during the healing process. Dressings which are used during the sensitive regeneration phase should be designed such that they do not get caught in the wound and should be flexible, and their contact surface with the wound should be soft. The dressings should also be able to absorb excess wound secretion or allow wound secretion to pass through to an absorptive body which is applied over the dressing or which is included in the dressing.

Examples of dressings which are used on wounds in the regeneration phase are ointment-saturated compresses composed of gauze or nylon fabric, where appropriate in combination with an absorptive body. While these dressings have less of a tendency to get caught in the wound than do conventional fibre dressings, they nevertheless have a number of drawbacks; for example, they frequently do get caught in the wound despite the impregnation and give rise to tissue damage.

The EP patent 0 261 167 B1 describes a dressing which is markedly superior. This patent relates to a dressing for discharging wounds which comprises a hydrophobic layer which directly contacts the wound during use and which is permeable to liquid. This dressing is characterized by the fact that the hydrophobic layer consists of a soft and elastic gel, preferably in the form of a silicone gel, which is applied to a lattice-like reinforcement enclosing all the lattice parts but leaving through-holes. A dressing of this type has to all intents and purposes eliminated the problem of adherence in the wound.

Another problem which can interfere with wound healing, and render it impossible, is the presence or growth of bacteria in the wound region. The presence of copious numbers of bacteria can also result in an odour which can constitute a social handicap and an intractable affliction. The wound surface can also constitute the entry point for more serious systemic infections which require hospitalization and a relatively long period of convalescence and can lead to death in some instances.

Several of the bacterial species which are found in wounds are difficult to treat with antibiotics. The treatment is made yet more difficult by the fact that the high and prolonged antibiotic pressure to which patients with wound infections are frequently subjected selects resistant strains. These multiresistant wound bacteria, which have constituted a serious threat in recent years and led to pronounced clinical problems, include methicillin-resistant Staphylococcus aureus (MRSA), vancocin-resistant enterococci (VRE) and completely resistant Pseudomonas aeruginosa.

Patients suffering from diabetes are a group which is very vulnerable from the point of view of wound infections. The number of diabetic patients is constantly increasing, with this applying to extensive areas of the world. In a general manner, the disease impairs the ability of the body to defend itself against infections, and changes in touch perception and circulation result in it being easy for wounds to arise. In addition, the higher contents of glucose in the blood and tissues constitute a favourable substrate for most bacteria. The beta-haemolytic streptococci constitute a group of bacteria which is especially favoured by the good access to sugar. Streptococci are genetically very well equipped with regard to sugar-degrading enzymes and their niche region is therefore located in the mouth, where sugar contents are highest. These bacteria also readily give rise to infections in diabetics which are relatively severe. This also applies to group B streptococci which are normally bacteria of relatively low virulence in adults. Chronic foot and leg wounds are a special problem in diabetics, with broad spectrum antibiotics being required over relatively long periods in order to avoid amputations. There is a great risk of this care resource-demanding group hastening the development of resistance and decreasing the time interval up until the post-antibiotic era. These patients therefore have a very great need for alternative forms of treatment.

As is evident from the above examples, the problems involved in treating wound infections have not decreased, but have if anything increased, despite new and improved wound care products and treatment methods.

### DISCLOSURE OF THE INVENTION

The present invention has resulted in a wound care product whose use greatly improves the possibility of healing infected wounds.

According to the invention, a wound care product which is of the type mentioned at the outset and which comprises a substance which inhibits the growth of bacteria in wounds is characterized in that the substance is xylitol.

According to one embodiment, the invention is characterized in that the said wound care product comprises of a gel into which xylitol is introduced. According to a modified embodiment, the invention is characterized, in this connection, in that the said gel is a silicone gel.

According to another embodiment, the invention is characterized in that the wound care product consists of or comprises a layer which directly contacts the wound during use and which is permeable to wound liquid, in that the layer consists of a gel and a lattice-like reinforcement, in that the gel is applied enclosing all the lattice parts but leaving through-holes in the layer formed by the gel and the reinforcement, and in that xylitol has been introduced into the gel. According to one embodiment, the invention is characterized, in this connection, in that the said layer is hydrophobic and does not adhere to discharging wounds. According to one embodiment, the gel is a silicone gel.

According to another embodiment, the invention is characterized in that the wound care product comprises a dressing and in that xylitol has been applied to a support which is included in this dressing. According to one embodiment, the said support is gauze. According to another embodiment, the support is a non-woven fabric.

According to another embodiment, the support is a polymer foam possessing open pores. According to another embodiment, the support is a water-soluble polymer foam.

According to another embodiment, the said wound care product is in the form of a dressing and is characterized in that the said dressing contains an absorbent layer, for taking up secreted wound liquid.

According to one embodiment of a method for manufacturing a wound care product, the invention is characterized in that xylitol is applied to the support in the form of a solution. According to one embodiment of the said method, the support is then dried.

The invention also relates to the use, in wound care products, of a substance which inhibits the growth of bacteria in wounds. According to the invention, this use is principally characterized in that the said substance is xylitol. According to one embodiment of the use, the said substance is in powder form. According to another embodiment of the use, the said substance is included in a gel. According to another embodiment of the use, the said substance is included in a liquid solution. According to another embodiment of the use, the said substance is included in an ointment. According to yet another embodiment of the use, the said substance is included in a paste.

The invention also relates to a wound dressing in the form of a plaster containing a substance for inhibiting the growth of bacteria in wounds. In this connection, the invention is characterized in that the said substance is xylitol.

According to another embodiment, the invention consists of using xylitol for producing a composition which has a growth-inhibiting effect on bacteria in wounds.

According to one embodiment, this use is characterized in that the said composition comprises a liquid solution which contains xylitol.

According to one embodiment, the wound care product is sterile in its entirety and is packaged in a sterile manner.

As is evident from the above, the essence of the invention is the use of xylitol for inhibiting the growth of bacteria which are found in wounds.

Xylitol (birch sugar) is a natural carbohydrate which occurs in free form and in small quantities in particular plant parts in trees, vegetables and fruit, inter alia, and in human intermediary metabolism. Xylitol has been known in organic chemistry since at least the 1890s. German and French research workers were the first to manufacture xylitol chemically more than 100 years ago. Xylitol was finally characterized and purified during the 1930s. Despite xylitol having a relatively long history from the chemical point of view, it was regarded for a long time as being one of several sweet carbohydrates. However, the lack of sugar during the second world war in a number of countries increased the interest in xylitol. It was not until research workers studied its insulin-independent nature that its biological properties began to be understood and, up until the 1970s, xylitol was used in a number of countries as a sweetener for diabetics, in connection with parenteral nutrition, i.e. nutrition given directly via a blood vessel, or in connection with treating insulin coma. The use of xylitol in connection with dentistry only began during the 1970s and the first xylitol-containing chewing gum for controlling caries was launched in Finland in 1975.

Xylitol is a sugar alcohol of the pentitol type (CH₂OH(CHOH)₃CH₂OH) having five carbon atoms and five hydroxyl groups. It can therefore be termed pentitol. Xylitol belongs to the polyalcohols (polyols) which are not sugars in the strict sense. However, they are biochemically related to sugars due to the fact that they are manufactured from, and can be converted into, sugars. In addition, some chemical works of reference define sugars as being crystalline, sweet carbohydrates, which is a category which covers xylitol.

No mutagenic properties, i.e. increase in the natural mutation frequency, have been detected when this sugar alcohol has been used in bacteriological tests. (Batzinger et al; Saccharin and other sweeteners: mutagenic properties. Science 1977; 198:944-946). In addition, a number of national and international authorities have assessed its toxicity as being so low that there is no limit value for an acceptable daily intake (Mäkinen; Dietary prevention of dental caries by xylitol - clinical effectiveness and safety; J. Appl. Nutr. 1992; 44:16-28).

The bacterial effect which has first and foremost been studied and documented is the dental effect, which is largely an effect of the structure of this compound. Most dietary polyols are hexitols. From the evolutionary point of view, it has not, therefore, been advantageous for bacteria to break down anything other than the hexitols. For this reason, most bacteria are not equipped enzymatically for using pentitols for their growth.

Adding xylitol (1-10%) to nutrient-rich medium therefore reduces the growth of the bacteria which are most frequently found in conjunction with wound infections, i.e. S. aureus (including MRSA), group A, B, C and G streptococci, enterococci (including VRE) and Pseudomonas aeruginosa, by a factor of up to approx. 1000, as has been shown by our own experiments, which are described in more detail below. These results are independent of the resistance of the bacteria to antibiotics.

The growth of alpha-streptococci in the oral flora is inhibited by the fact that xylitol is taken up by way of a fructose phosphotransferase system. The sugar alcohol accumulates in the bacterium without being able to be broken down and can be directly toxic (Trahan et al. Transport and phosphorylation of xylitol by a fructose phosphotransferase system in Streptococcus mutans. Caries Res. 1985; 19:53-63); however, if fructose is supplied, the situation is normalized, i.e. a competitive relationship then exists (Tapiainen et al. Effect of xylitol on growth of Streptococcus pneumoniae in the presence of fructose and sorbitol). This relationship is not equally obvious in the commonest wound bacteria. Xylitol may therefore interfere with different functions in the case of these microorganisms.

In addition to affecting growth, the uptake of xylitol also affects protein synthesis in alpha-streptococci (Hrimech et al. Xylitol disturbs protein synthesis, including the expression of HSP-70 and HSP-60, in Streptococcus mutans. Oral Microbiol. Immunol. 2000; 15:249-257). In addition to other effects, the production of stress proteins, which are required for the ability of the bacterium to adapt to an inimical environment, is altered. This increases the vulnerability of the bacterium.

A decrease in the production of glycocalyx, a sugar substance which increases the ability of the bacteria to adhere to, and colonize, the wound tissue, has been demonstrated in S. aureus (Akiyama et al. Actions of farnesol and xylitol against Staphylococcus aureus. Chemotherapy 2002; 48:122-128). The fact that xylitol decreases the adherence of bacteria has also been demonstrated in the case of intestinal bacteria which cause diarrhoea and bacteria which give rise to ear inflammations and in the case of yeast fungus (Naaber et al. Inhibition of adhesion of Clostridium difficile to Caco-2- cells. FEMS Immunol. Med. Microbiol. 1966; 14:205-209. Kontiokari et al. Antiadhesive effects of xylitol on otopathogenic bacteria. J. Antimicrob. Chemother. 1998; 41:563-565. Pizzo et al. Effect of dietary carbohydrates on the in vitro epithelial adhesion of Candida albicans, Candida tropicalis, and Candida krusei. New Microbiol. 2000; 31:63-71).

Adhesion and colonization represent the first phase for the bacteria in an infection process and are therefore of great significance for infection frequency. A clinical study in which xylitol reduced the number of ear inflammations in children by up to 40% demonstrates how important this can be (Uhari et al. Xylitol in preventing acute otitis media. Vaccine 2000; 19 Suppl. pp 144-147).

While intestinal bacteria are found first and foremost in conjunction with chronic, and not acute, wound infections, their significance in wound infections is not clear. Some intestinal bacteria can break down xylitol while others have to mutate in order to obtain this property. The mutations often occur at a certain price. Either the bacterium can only break down xylitol for a relatively short period or else the bacterium grows less well in its normal environment when the sugar alcohol is removed (Scangos et al. Acquisition of ability to utilize xylitol: disadvantages of a constitutive catabolic pathway in Escherichia coli. J. Bacteriol. 1978; 134:501-505. Inderlied et al. Growth of Klebsiella aerogenes on xylitol: implications for bacterial enzyme evolution. J. Mol. Evol. 1977; 9:181-190).

However, in the case of oral bacteria, there is a risk of xylitol-resistant strains being selected (Hrimech et al. 2000) when the xylitol is used over a long period. However, this type of bacteria is rarely involved in wound infections on the legs or feet.

Xylitol is only absorbed to a very slight extent by epithelial cells in the skin and, if anything, it extracts liquid due to its hyperosmolar nature. This does not affect the activity of endogenous substances, such as defensins, which possess antibacterial properties (Zabner at al. The osmolyte xylitol reduces the salt concentration of airway surface liquid and may enhance bacterial killing. Proc. Natl. acad. Sci. USA 2002; 97:11614-11619).

The high endothermal heat of xylitol in solution provides it with a cooling feeling on contact with mucous membrane or skin. Xylitol can also form complexes with calcium and other polyvalent cations. It is possible that these complexes contribute to increasing the absorption of calcium and can contribute to remineralization in regions of bone necrosis, a complication which is not entirely unusual in conjunction with deep leg and foot wounds. Like other sugar alcohols, xylitol has a protein-stabilizing effect as a result of protecting proteins in aqueous solution from denaturation, structural change and other damage, something which may be of significance in connection with wound healing.

As is evident from the above, a great deal has been written, since the 1970s, about the biological properties of xylitol, with the main emphasis being on the dental context.

Despite all the research and everything which has been documented with regard to xylitol over several decades, no one has, prior to the present invention, elucidated or perceived the fact that xylitol has great potential in the context of wounds and can constitute a powerful weapon for combating the presence and growth of the bacterial species which are most frequent and virulent in conjunction with wound infections, and that suitable wound care products can be produced for this purpose. In contrast to using antiseptics and toxic metal ions, this can take place without there being any risk of developing bacteria which are multiresistant to antibiotics or of there being any harmful effects on the tissue. Similarly, there are no risks of ecological disturbances or allergic reactions as in the case of the systemic use and local use, respectively, of antibiotics.

Xylitol can be successfully employed in many situations in connection with wound infections where previously known wound care products and treatment methods have been without effect or have had an adverse effect. As will be evident from the following description, our experiments have shown that xylitol is effective in combating the growth of MRSA, VRE and Pseudomonas aeruginosa, which bacteria cause intractable infections involving high medical treatment costs and long isolation times. Furthermore, our experiments show that xylitol is also effective against beta-haemolytic streptococci, including group B streptococci, which have previously been a frequent cause of relatively severe and invasive infections in conjunction with diabetic wounds.

As has been mentioned above, the biological effects of xylitol are also, naturally, well documented without it having been possible to demonstrate any negative effects.

### DESCRIPTION OF THE FIGURES

In that which follows, the invention will be described with reference to implemented tests which are shown on the attached drawings in which:
Figure 1 illustrates, in diagram form, the growth of S. aureus in a nutrient-rich medium without and, respectively, with the addition of different contents of xylitol.
Figure 2 illustrates, in diagram form, the growth of S. aureus (multiresistant) in a nutrient-rich medium without and, respectively, with the addition of different contents of xylitol.
Figure 3 shows, in diagram form, the growth of group B streptococci in a nutrient-rich medium without and, respectively, with the addition of different contents of xylitol.
Figure 4 shows, in diagram form, the growth of group G streptococci in a nutrient-rich medium without and, respectively, with the addition of different contents of xylitol.
Figure 5 shows, in diagram form, the growth of P. aeruginosa in a nutrient-rich medium without and, respectively, with the addition of different contents of xylitol.

### EMBODIMENTS

As is evident from the attached figures, implemented tests have demonstrated an exceptionally good effect as regards inhibiting the growth of common bacterial species which are found in wounds.

The experiments were carried out in the following manner.

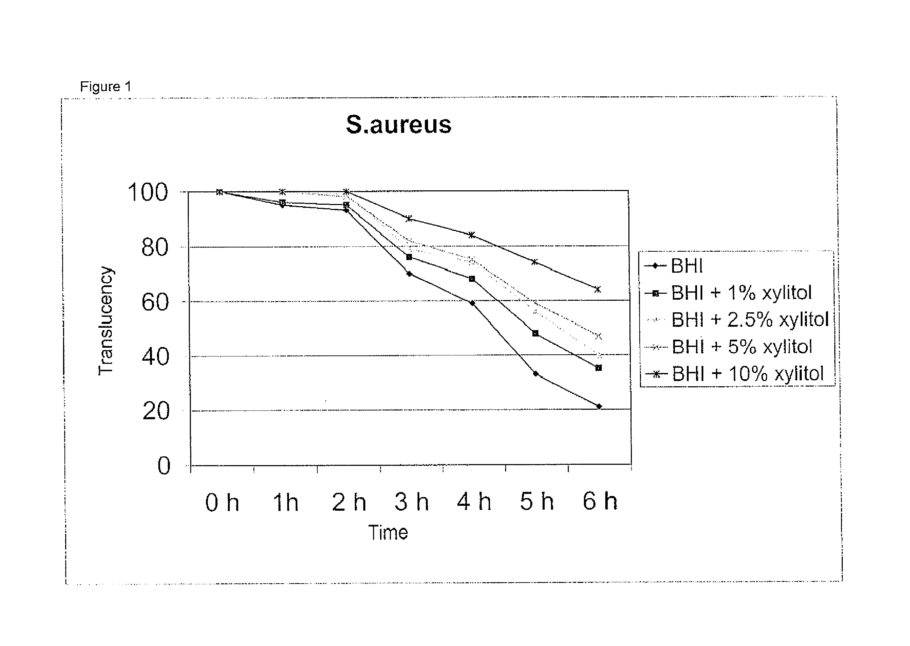

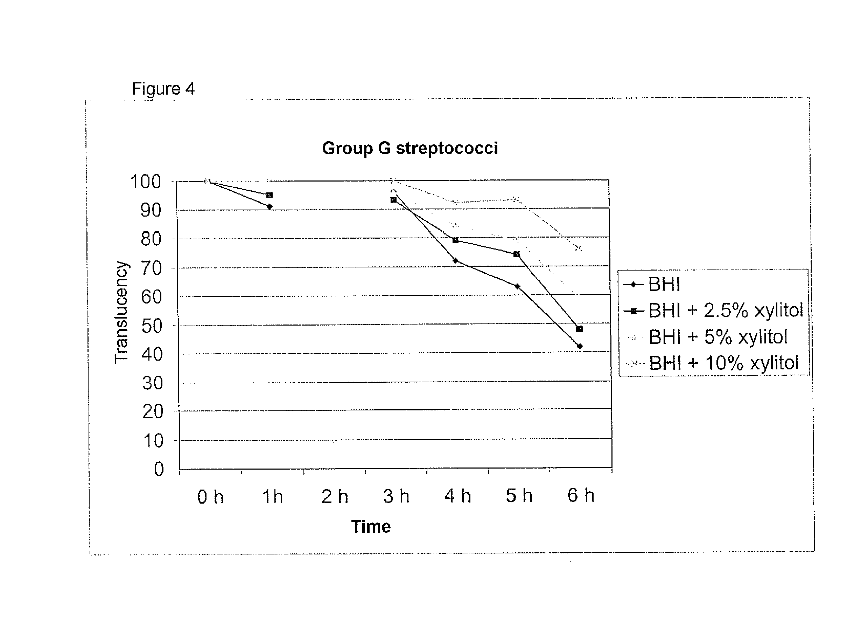

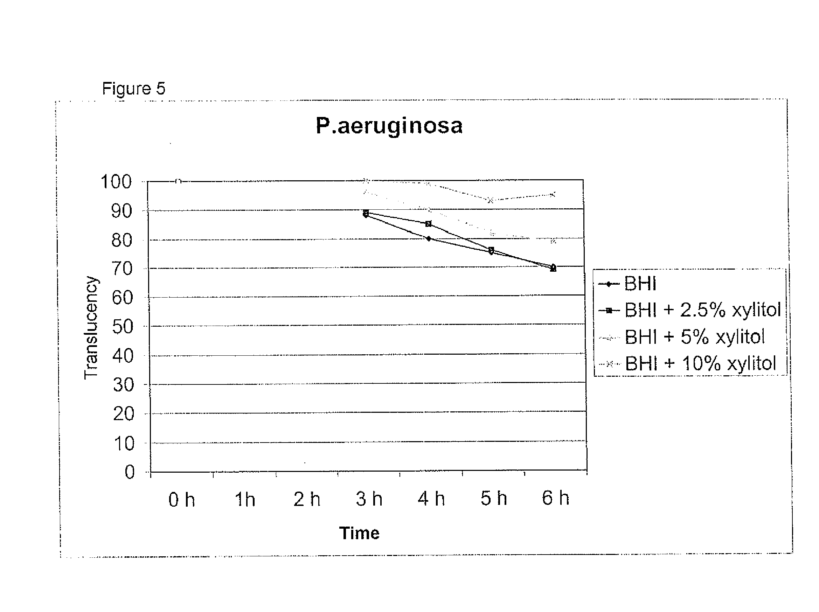

The bacterial strains were stored at -70°C. Immediately prior to each experiment, these strains were taken out and placed on a blood plate and incubated overnight at 35°C. The same incubator was used in all the experiments. After that, a loop of the colonies which had grown was incubated in brain heart infusion (BHI) broth for 18 hours. 100 µl of this broth were inoculated into 3-5 ml of fresh BHI broth with or without the addition of xylitol. Different contents of xylitol were added. As is evident from figures 1-5, the contents of xylitol were 1%, 2.5%, 5% and 10%.

The transmittance or translucency was measured from hour 0, and after that every hour, for 6 hours on a Biolog turbidimeter from the company Biolog Inc., Hayward, CA, USA. The growth of bacteria reduces the translucency, i.e. the lower the transmittance the greater the growth of the bacteria.

At 0 hours and at 6 hours, 100 µl of the broth were cultured and the number of live bacteria was counted. The transmittance value and the quantity of bacteria (colony forming units/ml) in tubes with and without xylitol were then compared.

In the graph shown in figure 1, it can be seen that the translucency of the bacterium S. aureus, i.e. what is termed the hospital bacterium, in BHI without added xylitol decreased gradually from 100 at the time point 0 to 20 at the time point 6 hours, indicating that the bacteria had grown very strongly. In the same graph, it can be seen that the translucency was still more than 60% after 6 hours in the nutrient solution to which 10% xylitol had been added, indicating that the bacterial growth had been very greatly moderated as compared with the solution without xylitol. As is furthermore evident from the curve for BHI containing 10% added xylitol, hardly any bacterial growth at all had occurred after 2 hours.

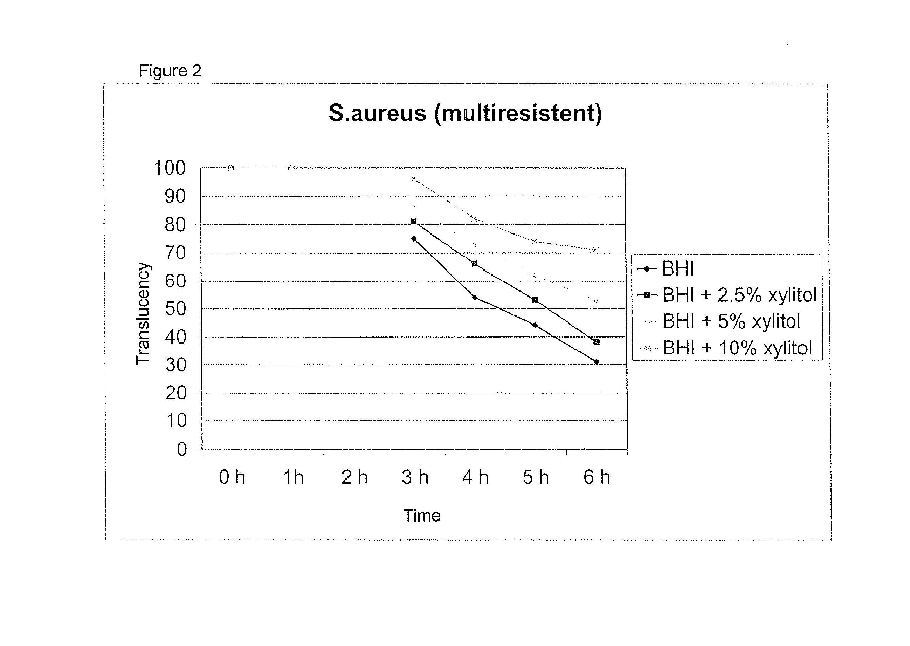

The graph in figure 2 shows curves where a Staphylococcus aureus variant was used with or without added xylitol. The bacterium shown in figure 2 is an MRSA strain exhibiting a very high degree of resistance and where only one or two antibiotic types are possible treatment alternatives. These drugs cost more than 1000 SEK per day as compared with normal prices of the order of 55-70 SEK per day. In addition, the multiresistance has resulted in patients infected with MRSA having to be cared for in separate rooms with special hygiene regulations and not being allowed to be moved randomly in the hospital. This is naturally very costly and is trying for the patient.

As is evident from figure 2, adding xylitol to the nutrient solution containing the bacteria results in the growth of the bacteria in the solution being inhibited very effectively. The growth is inhibited to an increasing degree as the amount of added xylitol increases. After 6 hours, the translucency in the presence of 10% added xylitol is more than 70% while the translucency for a solution without any added xylitol is only approx. 30%. In figure 2, no values were measured after 2 hours.

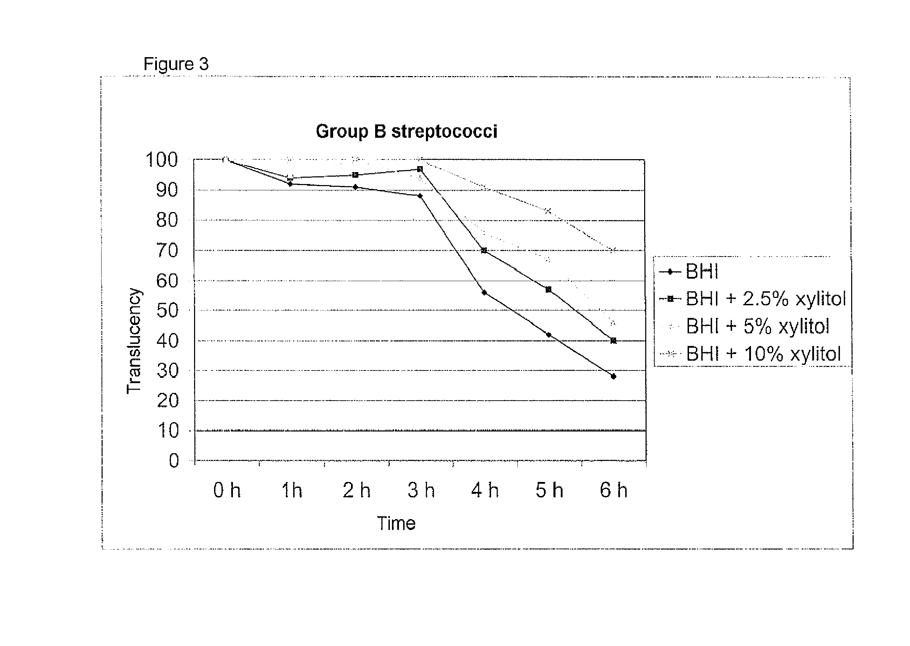

Figure 3 shows a graph corresponding to those shown in figures 1 and 2 but in this case for group B streptococci. This bacterium type is commonly found in severe diabetic wounds, in particular leg and foot wounds, which entail the risk of life-threatening infections and bone necrosis.

As can be seen from figure 3, the growth of these bacteria can be effectively inhibited by adding xylitol.

In a corresponding manner to that in figures 1-3, figures 4 and 5 show two further bacteria, i.e. group G streptococci and P. aeruginosa, which are found in wounds. As these graphs show, xylitol is very effective in inhibiting the growth of these bacterial strains as well.

According to the invention, wound care products comprising xylitol can be designed in different ways.

One way is to add xylitol in powder form to a silicone gel. This latter is a chemically crosslinked silicone gel (polydimethylsiloxane gel), for example a platinum-catalyzed 2-component addition-curing RTV silicone. Examples of gels which can be used are SiGel 612 from Wacker-Chemie GmbH, Burghausen, Germany, and MED-6430 from NuSil Technology, Carpinteria, USA. Examples of self-adhering gels are also described in GB-A-2 192 142, GB-A-2 226 780 and EP-A1-0 300 620. Other hydrophobic gels, such as hydrophobic polyurethane gels, are also conceivable.

According to one embodiment, xylitol in powder form can be added to, and mixed with, liquid silicone of the abovementioned types. After that, this mixture is adhesion-cured, for crosslinking the polymer lattice, at a temperature of 90-130°C. The silicone gel containing xylitol can be used as a wound care product, expediently in combination with an outer wound dressing.

Xylitol is available commercially in powder form, approximately of the same grain size as granulated sugar. According to one embodiment, the xylitol powder can be ground so as to obtain a more fine-grained powder having a higher specific surface. Admixing in silicone results in an increase in the surface exposed and this will increase the release of xylitol from a silicone gel containing xylitol as compared with the same silicone gel and xylitol having a larger grain size. The grain size of the xylitol can thus be used to vary the rate of release from the silicone gel containing xylitol.

Alternatively, the xylitol can firstly be dissolved in water; after that, a suspension of silicone and the xylitol solution is prepared, with the suspension then being cured.

A silicone gel containing xylitol in particle form or having been added in solution can be used for producing a wound care product which comprises a layer which contacts the wound directly during use, which is permeable to wound liquid and which comprises the said silicone gel containing xylitol and a lattice-like reinforcement. The gel is applied so that it encloses all the lattice parts but leaves through-holes in the layer formed by the gel and the reinforcement. A dressing which is of this type, but which does not contain xylitol, is described in our EP patent 0 261 167 B1, the entire content of which is hereby incorporated by reference.

The invention is not limited to the above-described embodiments and a number of modifications are possible within the scope of the subsequent patent claims.

For example, the dressing can contain xylitol, in solution or in particle form, which is applied to, or introduced into, a support, such as a polymer foam possessing open pores. An example is polyurethane foam of the Hypol® type from Hampshire Chemical Corporation, Lexington, Massachusetts, USA.

Other examples of supports are gauze, hot-melt adhesive and nonwoven fabric.

The wound care product is sterilized in its entirety and packaged in a sterile manner.

## Claims

1. Wound care product, comprising a substance which inhibits the growth of bacteria in wounds, which wound care products comprises a layer, which directly contact the wound during use and which is permeable to wound liquid, **characterized in that** the substance is xylitol, **in that** said wound care product comprises a gel into which xylitol has been introduced and **in that** said gel is arranged in the layer.

2. Wound care product according to claim 1, **characterized in that** said gel is a hydrophobic and soft gel.

3. Wound care product according to claim 2, **characterized in that** said gel is a silicone gel.

4. Wound care product according to claim 2, **characterized in that** said gel is a polyurethane gel.

5. Wound care product according to any of the preceding claims, **characterized in that** said layer is a polymer foam with open pores.

6. Wound care product according to claim 3 or 4, **characterized in that** the layer comprises a lattice-like reinforcement, **in that** the gel has been applied so that it encloses all the lattice parts but leaves through-holes in the layer formed by the gel and the reinforcement.

7. Wound care product according to claim 6, **characterized in that** said lattice-like reinforcement is elastic.

8. Wound care product according to claim 7, **characterized in that** the reinforcement comprises a soft, flexible and elastically stretchable network of textile material.

9. Wound care product according to any of the preceding claims and in the form of a dressing, **characterized in that** said dressing contains an absorbent layer for taking up secreted wound liquid.

10. Use of a substance in wound care products, which substance reduces the growth of the bacteria which are most frequently found in conjunction with wound infection, including MRSA, A,B,C and G streptococci, VRE and Pseudomonas aeruginosa, which wound care products comprises a layer, which directly contact the wound during use and which is permeable to wound liquid, **characterized in that** said substance is xylitol, **in that** the xylitol is arranged in a gel and **in that** said gel is arranged in said layer.
